# EUROPÄISCHE PATENTSCHRIFT

(11) **EP 1 143 785 B1**
(45) Veröffentlichungstag und Bekanntmachung des Hinweises auf die Patenterteilung: **28.07.2004**
(21) Anmeldenummer: 00902552.9
(22) Anmeldetag: 11.01.2000
(51) Int. Cl.: A01G 7/04, A01H 3/02

(54) **VERFAHREN ZUM HERVORRUFEN ODER FÖRDERN EINER ANTHOCYANFÄRBUNG IN GRUNDSÄTZLICH ANTHOCYAN-BILDENDEN PFLANZEN UND/ODER FRÜCHTEN**
METHOD FOR INDUCING OR PROMOTING AN ANTHOCYANIN COLORATION IN PLANTS AND/OR FRUIT WHICH BASICALLY PRODUCE ANTHOCYANIN
PROCEDE DE PRODUCTION OU DE STIMULATION D'UNE COLORATION ANTHOCYANIQUE DANS DES PLANTES OU DES FRUITS PRODUISANT FONDAMENTALEMENT DE L'ANTHOCYANE

(30) Priorität: 11.01.1999 DE 19900616
(43) Veröffentlichungstag der Anmeldung: 17.10.2001
(73) Patentinhaber: Scherer, Günther, 30827 Garbsen-Beerenbostel (DE)
(72) Erfinder: Scherer, Günther, 30827 Garbsen-Beerenbostel (DE)
(74) Vertreter: Läufer, Martina, Dipl.-Chem. rer. nat.
(86) Internationale Anmeldenummer: PCT/DE2000/000068
(87) Internationale Veröffentlichungsnummer: WO 2000/041557

(56) Entgegenhaltungen:
- FR-A- 2 542 567
- ARAKAWA, O.: "Photoregulation of Anthocyanin Synthesis in Apple Fruit un UV-B and Red Light" PLANT CELL PHYSIOLOGY, Bd. 29, Nr. 8, 1988, Seiten 1385-1389, XP000911138
- ARAKAWA ET AL.: "Relative effectiveness and interaction of ultraviolet-B, red and blue light in anthocyanin synthesis of apple fruit" PHYSIOLOGIA PLANTARUM, Bd. 64, 1985, Seiten 323-327, XP000911121 Copenhagen
- DATABASE BIOSIS [Online] BIOSCIENCES INFORMATION SERVICE, PHILADELPHIA, PA, US AN PREV199698593748, 1995 YANG YONG-JOON: "Changes in carotenoid pigments in the peel of "Fuji" apple fruit during cold CA storage." XP002139108
- PATENT ABSTRACTS OF JAPAN vol. 013, no. 388 (C-630), 28. August 1989 (1989-08-28) & JP 01 137925 A (ATSUSHI MAKINAE), 30. Mai 1989 (1989-05-30)

## Beschreibung

### Gebiet der Erfindung:

Die Erfindung betrifft ein Verfahren zum Hervorrufen einer Anthocyanfärbung in grundsätzlich Anthocyanbildenden Pflanzen und/oder Früchten.

### Hintergrund der Erfindung:

Eines der wichtigsten Ziele der Fruchtproduktion ist die möglichst lange Lagerung von Früchten, die anschließend noch attraktiv aussehen, gut schmecken und gesund sein müssen. Aus diesem Grund sind Äpfel als Früchte in gemäßigten Breiten so wichtig, da die meisten Sorten diese Bedingungen erfüllen. Um dieses Ziel zu erreichen, erfolgt die Lagerung von Äpfeln heutzutage in sog. ULO-Lagern ("Ultra Low Oxygen") oder CA-Lagern ("Controlled Atmosphere"), in denen bei 0°C ein erhöhter Kohlendioxid- und ein stark verminderter Sauerstoffgehalt vorhanden ist. Stoffwechsel und Nachreife werden so verhindert und Lagerung bis zu 6 Monaten und mehr wird erreicht. Früher wurde nur die Temperatur reguliert, was zu kürzeren Lagerzeiten führt. Für den Verbraucher ist die Stärke der roten Pigmentierung (Färbung) der Rinde von Äpfeln ein wichtiges Kriterium für deren Qualität. Die Rotfärbung bestimmt damit den Wert der Äpfel am Markt.

Daher wird es als nachteilig empfunden, daß bei Früchten immer schon die Rotfärbung innerhalb eines Baumes stark variierte, und die im Schatten gewachsenen Früchte als qualitativ geringer eingestuft werden, und zwar vorwiegend wegen der Farbe. Außerdem gibt es etliche bekannte und wohlschmeckende Sorten, die schlecht rot werden und deswegen weniger beliebt sind, als sie vielleicht sein könnten, z.B. Cox Orange. Erfahrungen beim Verkauf verschiedener Sorten gehen eindeutig dahin, daß rote Farbe über allen anderen Auswahlkriterien steht. Wichtige Sorten, die überhaupt nicht rot werden, sind Golden Delicious (Europa und weltweit) und Granny Smith, der meist auf der Südhalbkugel (Neuseeland) produziert wird.

Deshalb ist die Erzeugung der Rotfärbung der Äpfel ein ständiges Ziel des Apfelbauern.

Obwohl der Mechanismus der Pigmentierung der Apfelrinde noch nicht vollständig aufgeklärt ist, ist es bekannt, daß eine ausreichende Belichtung mit Sonnenlicht, aber auch künstlichem Licht (DE 3409796, WO 86/00492), die Pigmentierung verstärkt. Darüber hinaus ist bekannt, daß auch chemische Substanzen (FR 81 15845, EP 0 598 304) die Pigmentierung der Rinde von Äpfeln vorteilhaft beeinflussen können. Allerdings ist die Verwendung chemischer Substanzen nicht immer unbedenklich (FR 81 15845) bzw. zeigt nicht bei allen Apfelsorten den gewünschten Effekt, und die Methoden, bei denen Sonnenlicht eingesetzt wird, sind auf dessen Verfügbarkeit angewiesen.

Bekannte Verfahren zur künstlichen Belichtung setzen im allgemeinen weißes oder sonnenlichtartiges Licht ein, d.h. sie versuchen die natürliche Sonnenbestrahlung durch eine entsprechende Strahlung zu ergänzen oder zu ersetzen.

Ferner ist es aus der DE 34 09 796 A1 bereits bekannt, die Anthocyanbildung in Früchten und Pflanzen durch eine Kombination aus blauem und rotem Licht zu fördern. Die Auswahl des dort für die Bestrahlung gewählten Spektralbereiches bezieht sich darauf, daß für die Anthocyansynthese zwei fotochemische Reaktionen bekannt sind, nämlich eine energieschwache, rot/langwellig-rot, umsteuerbare (reversible) phytochrom gesteuerte Reaktion und eine Intensivbestrahlungsreaktion, die im blauem und im langwellig roten Bereich des sichtbaren Lichtspektrums am wirksamsten ist. Für diese bei der Anthocyanbildung beteiligten Fotoreaktionen wird das Phytochrom als Fotorezeptor diskutiert bzw. angenommen.

Die WO 86/00492 beschreibt ein Verfahren zur Kennzeichnung von Äpfeln, in dem die Äpfel mit einer lichtundurchlässigen Maske versehen werden und dann mit einer künstlichen Lichtquelle, z.B. mit einer weißes Licht emittierenden Leuchtstoffröhre ("fluorescent light source") bestrahlt werden.

Die Ansprüche des Marktes fordern heutzutage einerseits gut aussehende und optimal gerötete Früchte, wobei der Markt insbesondere der Anwendung künstlicher Mittel, wie z.B. der Aufbringung chemischer Substanzen, kritisch begegnet. Die Verwendung von Licht zur Herbeiführung der Rötung kommt dagegen ohne die Anwendung chemischer Mittel aus. Allerdings sind die bisher beschriebenen Verfahren, wie die Nachtunterbrechungsbehandlung vor der Ernte aufwendig und wenig effizient und die bekannten Bestrahlungsverfahren mit weißem, blauem oder rotem Licht sind langwierig und schon deshalb verbesserungsbedürftig. Eine Veränderung der Farbe anthocyangefärbter Früchte oder Pflanzenteile im Sinne einer Attraktivitätssteigerung dieser Früchte oder Pflanzen konnte bislang nicht erzielt werden.

Eine Förderung der natürlichen Anthocyanrotfärbung konnte beispielsweise wie in "PHYSIOL. PLANT 64, 323-327, 1985" beschrieben an roten Äpfeln (Jonagold) mit UV-B und dem Rotanteil von weißem Licht erzielt werden, oder wie in der FR-2 542 567 beschrieben, mit einer Kombination aus rotem und blauem Licht, wobei im letzteren Fall eine Behandlung über 30-45 Tage (an rotem Delicious, ohne Nachtbestrahlung) erforderlich war.

Der vorliegenden Erfindung liegt daher die Aufgabe zugrunde, ein Verfahren zum Hervorrufen oder Fördern einer Anthocyanfärbung in Pflanzen und/oder Früchten bereitzustellen, das sich durch eine besonders schnell erzielbare Wirkung auszeichnet und sich problemlos in die etablierten Schritte vom Anbau bis zum Verkauf integrieren läßt, wobei insbesondere die Rotfärbung von Pflanzen(teilen) oder Früchten ermöglicht werden soll, die normalerweise keine rote Farbe entwickeln, z.B. von sog. grünen Apfelsorten.

### Zusammenfassung der Erfindung

Überraschend wurde jetzt gefunden, daß UV-B Licht, und Licht, das sich aus einer Mischung von weißem Licht und Licht aus dem spektralen Bereich des UV-B zusammensetzt, die Anthocyanbildung von Pflanzen und/oder Früchten nicht nur fördert, sondern ggf. erst induziert. Bei einer Verwendung von UV-B-Licht zusammen mit weißem Licht soll der UV-B-Anteil höher als in Sonnenlicht sein, wobei davon ausgegangen wird, daß der dieser (im Mittel) 2,5% beträgt.

### Detaillierte Beschreibung der Erfindung

Das erfindungsgemäße Verfahren läßt sich im Prinzip auf alle anthocyanbildenden Strukturen von Pflanzen (z.B. Blüten und Blätter) bzw. deren Früchte anwenden. Anthocyane verursachen gelbe, orange, rote und blauviolette sowie blaue Farbtöne, wobei es sich um verschiedene Stoffgemische handelt, die in den Vakuolen gespeichert werden. Fast alle oberflächlich liegenden Zellen (Epidermis) von den oberirdischen Organen der Pflanzen speichern Anthocyane besonders gut, sind aber bei weitem nicht immer bunt, sondern oft mit farblosen, nur UV-Licht absorbierenden Anthocyanen gefüllt. Die gelben, gelbroten und roten Anthocyane sind chemisch etwas einfacher gebaut als die blauen. Manche Pflanzen können keine roten und blauen, manche keine blauen Anthocyane bilden. Anthocyane sind auch die Farbstoffe, die Blätter (z.B. Blutbuche, Buntnessel und viele Zierpflanzen) färben. Nicht alle gelben/roten Früchte/Blüten sind durch Anthocyane gefärbt, roter und gelber Paprika z.B. durch Carotinoide, die biosynthetisch völlig anders entstehen.

Bevorzugtermaßen wird das erfindungsgemäße Verfahren zum Hervorrufen der Rotfärbung (der Ausbildung roter Anthocyane), bei Früchten eingesetzt.

Wichtige Früchte, die durch Anthocyane rot werden, wie Äpfel, Birnen, Pfirsiche, Nektarinen, Pflaumen, Kirschen (alle Rosengewächse), Blaubeeren und Preiselbeeren, fallen in das Gebiet der Erfindung. Bevorzugtermaßen wendet man das erfindungsgemäße Verfahren bei Birnen und Äpfeln, insbesondere bei Äpfeln an.

Erfindungsgemäß wird die Rotfärbung von Früchten, insbesondere Äpfeln gefördert, die sich normalerweise nicht rot färben. Dies erreicht man durch die Bestrahlung mit UV-B-Licht oder einer Mischung aus UV-B-Licht und weißem Licht und wurde erfolgreich beispielsweise an den folgenden Apfelsorten durchgeführt.: *Golden Delicious* (*grün*), *Zitronenapfel, Granny Smith* und Mutsu.

Bei der Verwendung von UV-B-Licht setzt man bevorzugtermaßen Lichtquellen ein, deren Strahlungsflußanteil im Bereich von 280-315 nm bezogen auf den Gesamtstrahlungsfluß von 100-780 nm (Φ_{280-315 nm} /Φ₁₀₀₋₇₈₀ₙₘ; jeweils in Watt gemessen) nicht unter 10%, weiter bevorzugt nicht unter 20% liegt. In den derzeit bevorzugten Ausführungsbeispielen liegt der Wert für Φ₂₈₀₋₃₁₅ₙₘ /Φ₁₀₀₋₇₈₀ ₙₘ bei mindestens 30%, insbesondere bei mindestens 45%. Höhere Werte (z.B. mindestens 70 oder mindestens 90%) sind im Hinblick auf die Energieausbeute noch günstiger. Lampen mit solchen höheren Strahlungsflußanteilen im Bereich von 280-315 nm sind in der Regel aber teurer. Somit kann man mit günstigen käuflich erhältlichen UV-B-Lampen (z.B. TL 40W/12 der Fa. Philipps), deren Wert (ca. 57%) für Φ_{280-315 nm}/Φ_{100-780 nm} bei oberhalb von 45% liegt, bereits äußerst wirtschaftlich arbeiten.

Analog verwendet man vorzugsweise. Weißlichtquellen, deren Strahlungsflußanteil (Φ_{400-510 nm} /Φ_{100-780 nm}) bzw. (Φ_{400-780 nm} /Φ_{100-780 nm}) im Bereich von 400-510 nm bzw. 400-780 nm bezogen auf den Gesamtstrahlungsflusses von 100-780 nm bei mindestens 10%, insbesondere mindestens 20 bis 30%, am stärksten bevorzugt mindestens 45% liegt. Da Lichtquellen für weißes Licht mit Strahlungsflußanteilen von mindestens 70%, insbesondere mindestens 90% relativ günstig käuflich zu erwerben sind, ist das Arbeiten mit solchen Lichtquellen noch stärker bevorzugt.

Bei der Optimierung der Verfahrensbedingungen für die Bestrahlung spielen insbesondere die Zahl und Art der eingesetzten Lichtquellen, deren Leistung, deren Anordnung und Abstand relativ zu den Früchten, die Bestrahlungsdauer, die Temperatur und eine etwaige Nachlagerung unter Kühlung eine Rolle. Generell sollte die Bestrahlung mit Licht in dem oben bereits näher spezifizierten Wellenlängenbereich mit einer solchen Intensität und über eine solche Zeitdauer durchgeführt werden, daß der gewünschte Effekt erzielt wird. Der Fachmann kann die hierfür geeigneten Parameter - je nach den zur Verfügung stehenden Lichtquellen und der geometrischen Anordnung - experimentell ohne größeren Aufwand feststellen.

Üblicherweise verwendet man pro Lichtsorte 1-8, bevorzugtermaßen 1-4, insbesondere 2 Lichtquellen.

Die Anordnung der Lichtquellen stellt bevorzugtermaßen sicher, daß die Pflanze(n) bzw. die Frucht (Früchte) genau dort bestrahlt wird (werden), wo sich das Anthocyan bilden soll. Besonders bevorzugt ist die Anordnung zweier Lichtquellen pro Lichtsorte oberhalb der Pflanze (Frucht) bzw. der Pflanzen (Früchte). Bevorzugtermaßen ordnet man sowohl die Lichtquelle als auch die zu bestrahlenden Früchte (Pflanzen) in einem Gehäuse bzw. Behälter (insbesondere mit spiegelnden Oberflächen) an.

Der Abstand zwischen der (den) Lichtquelle(n) und den einzelnen Pflanzen (Früchten) beträgt bevorzugtermaßen bis zu 300 cm, insbesondere 25 bis 100 cm, bevorzugt 60 bis 80 cm. Man kann jedoch auch mit geringeren oder größeren Distanzen arbeiten, wenn man die anderen Verfahrensparameter (z.B. spektraler Strahlungsflußanteil und Leistung der Lampe, Bestrahlungsdauer) entsprechend anpaßt. Beispielsweise kann man einen größeren Abstand zwischen Früchten (Pflanzen) und Lichtquelle(n) durch eine höhere Leistung der Lichtquelle(n) oder durch einen höheren Strahlungsflußanteil derselben kompensieren.

Die Leistung der eingesetzten Lichtquellen liegt üblicherweise im Bereich bis zu 100W (20-100W), bevorzugtermaßen 36-60W pro Lichtquelle. Wegen Verlusten durch Wärme und Strahlung in "unerwünschten" Spektralbereichen wird in der Regel jedoch nur ein Bruchteil dieser Leistung im "gewünschten" Spektrenabschnitt abgegeben. Beispielsweise liegt bei der käuflich erhältlichen UV-B-Leuchstofflampe TL 40W/12 der Fa. Phillips die im Bereich von 280-315 nm abgegebene Leistung bei 5,1 W. Bei der Wahl einer geeigneten Lichtquelle ist ferner zu beachten, daß eine höhere Leistung bei ansonsten identischen Verfahrensparametern nicht automatisch eine beschleunigte Anthocyanbildung mit sich bringt, da es zu Sättigungseffekten kommen kann. In einigen Beispielen wurde eine Wattleistung von 10 bis 20 W/m² gemessen.

Bevorzugtermaßen arbeitet man im erfindungsgemäßen Verfahren mit den folgenden Lichtintensitäten, wobei sich die angegebenen Werte auf die Lichtintensität (in µEs⁻¹m²) an der Pflanze bzw. Frucht und auf den (die) Wellenlängenbereich(e) der jeweiligen Lichtsorte(n) beziehen.
UV-B: mehr als 0,5; stärker bevorzugt mehr als 1,0; insbesondere 10-20;
UV-B/Weiß: mehr als 0,75; stärker bevorzugt mehr als 1,5; insbesondere 15-20;

Für eine Mischung aus UV-B und weiß beträgt das Verhältnis bevorzugtermaßen 1/20 bis 10/1.

Es ist bevorzugt, die Bestrahlung über einen Zeitraum zwischen 6 h und mehreren Tagen, insbesondere 12 bis 72 h, stärker bevorzugt 12 bis 36, am stärksten bevozugt 12 bis 24 h durchzuführen. Bei der Bestrahlung von Früchten hängt die Wahl der Bestrahlungsdauer u.a. davon ab, ob die Früchte frisch geerntet sind oder bereits gelagert wurden. Frisch geerntete Früchte sprechen bei UV-B-Licht, ggf. gemischt mit weißem Licht, in der Regel stärker an als bereits längere Zeit gelagerte Früchte (z.B. mehr als 100 Tage, insbesondere mehr als 1 Jahr), so daß man innerhalb von 72h den Endwert der Rotfärbung erreicht. Bei bereits länger gelagerten Früchten können auch längere Bestrahlungszeiten als 72h zum Erreichen des Endwerts erforderlich sein. Der geeignete Bestrahlungszeitraum läßt sich durch Beobachtung der Pflanzen (Früchte) leicht ermitteln.

Auch die Temperatur hat auf die Anthocyanbildung einen Einfluß. Üblicherweise bestrahlt man bei Temperaturen von 5 bis 25°C, bevorzugtermaßen bei 14 bis 19°C (insbesondere 15 bis 18°C), wobei die Bestrahlung in einer Klimakammer häufig besonders vorteilhaft ist. Bei diesen Temperaturen werden auch das Aussehen und der Geschmack der Äpfel so wenig wie möglich beeinträchtigt. Erfindungsgemäß bewährt hat sich das Bestrahlen bei 17°C (vorzugsweise in einer Klimakammer). Eine Regelung der Luftfeuchtigkeit in der Klimakammer ist nicht erforderlich, kann aber dazu beitragen, die Früchte "frisch" zu halten.

Man kann das erfindungsgemäße Verfahren auf sich noch am Strauch oder am Baum befindende Früchte anwenden, z.B. als Nachtunterbrechungsbehandlung. Bei Baumfrüchten, z.B. Äpfeln und Birnen, ist es jedoch aus wirtschaftlichen Erwägungen bevorzugt, die Früchte zunächst zu ernten. Die Früchte können dann entweder im frischen Zustand oder nach einer frei wählbaren Zeit der Lagerung bestrahlt werden.

Insbesondere, wenn man "frische" Früchte bestrahlt, ist es bevorzugt, die bestrahlten Früchte nach der Bestrahlung im Dunkeln zu lagern. (Ob eine Frucht "frisch" ist oder nicht, hängt entscheidend von den Lagerbedingungen und der Sorte ab, so daß die hier beschriebene Verfahrensvariante nicht nur auf frisch geerntete Früchte, sondern bevorzugtermaßen auch auf bis zu 1 Jahr, insbesondere auf bis zu 100 Tage gelagerte Früchte angewendet werden kann). Während dieser Nachlagerung beobachtet man oft trotz Dunkelheit eine Anthocyanbidung, die diese Variante des erfindungsgemäßen Verfahrens besonders vorteilhaft macht. Bevorzugtermaßen kombiniert man eine Bestrahlung über einen Zeitraum von 12 bis 72h, vorzugsweise 12 bis 36h, insbesondere 12 bis 24h mit einer Nachlagerung im Dunkeln über mindestens 2 bis 7 Tage. Eine über 10 Tage hinausgehende Nachlagerung ist jedoch problemlos möglich.

Die Nachlagerung kann in einem Temperaturbereich von 0°C bis ca. 30-35°C durchgeführt werden, wobei im Hinblick auf die angestrebte Frischhaltung der Früchte Temperaturen von 0-10°C bevorzugt sind. Überraschenderweise wurde festgestellt, daß ein frisch gepflückter Apfel, der 1 Tag bestrahlt und dann nachgelagert wurde (z.B. 1 Tag Bestrahlung mit UV-B oder einer Mischung aus UV-B und weißem Licht, dann 7tägige Nachlagerung im Kühlschrank bei 4°C), eine stärkere Rotfärbung zeigte als ein frisch gepflückter Apfel, der 3 Tage unter denselben Bedingungen bestrahlt wurde und nicht nachgelagert wurde. Zur Nachlagerung im Dunkeln eignen sich auch die bereits erwähnten ULO-Lager oder CA-Lager mit ihren typischerweise bei 0°C liegenden Lagertemperaturen.

Nach dem erfindungsgemäßen Verfahren ist es auch möglich, die Pflanzen und/oder Früchte mit einer Aussparung der Anthocyanfärbung in einer beliebig zu wählenden Form zu versehen, indem man vor der Bestrahlung eine lichtundurchlässige Abdeckung mit dieser Form auf die nicht oder wenig pigmentierten Pflanzen und/oder Früchte aufbringt und diese Abdeckung nach der Bestrahlung wieder entfernt.

Damit ergibt sich nach dem erfindungsgemäßen Verfahren die Möglichkeit, ein im Prinzip bekanntes Verfahren zur Aufbringung von Zeichnungen und Schriftzügen auf die Oberfläche von Früchten, insbesondere Äpfeln wesentlich zu verbessern, da das neue Verfahren auch auf ansonsten grünbleibende Fruchtsorten angewandt werden kann. Ein weiterer Vorteil liegt darin, daß man diese Variante des Verfahrens auch noch lange Zeit nach der Ernte durchführen kann, wobei Belichtungszeiten von nur 2 Tagen genügen können, um eine deutlich sichtbare Anthocyanbildung hervorzurufen.

Die Fruchtoberfläche wird so z.B. zu einer neuen Werbefläche. Man kann auch an Weihnachtsmotive, Firmenlogos, Vornamen, Sprüche aller Art usw. denken, die so auf die Fruchtoberfläche aufgebracht werden können. Dies bereichert den normalen Obstverkauf um eine zusätzliche Möglichkeit. Da das Muster sehr schnell und unabhängig von der Sorte aufgebracht werden kann, bietet die Erfindung mit ihrer schnellen und einfachen Methode zur nachträglichen Rötung von Früchten, insbesondere Äpfeln, einen entscheidenden Vorteil gegenüber bekannten Verfahren. Das erfindungsgemäße Bestrahlungsverfahren ermöglicht es auch noch spät nach der Ernte kurzfristig auf Bestellung durch Aufbringen von Aufklebern und anschließende Bestrahlung ein Motiv auf die Oberfläche der Früchte aufzubringen.

Mit dem erfindungsgemäßen Verfahren läßt sich insbesondere beim Einsatz von spektralem UV-B oder einer Mischung aus UV-B und weißem Licht die Anthocyanbildung gegenüber herkömmlichen Verfahren beschleunigen.

Ein entscheidender Vorteil des erfindungsgemäßen Verfahrens bei der Bestrahlung von Pflanzen und/oder Früchten ist es, daß es ohne großen technischen Aufwand schnell durchgeführt werden kann und sich problemlos in die bestehenden Verfahrensschritte vom Anbau bis zum Verkauf eingliedern läßt. Bei Früchten, insbesondere Äpfeln ist es von großem Vorteil, daß sich das Verfahren in etablierte Lagerungsschritte integrieren läßt. Somit kann man die Früchte, insbesondere Äpfel ohne Frischeverlust bestrahlen und dann wie herkömmlich unter ULO- oder CA-Bedingungen lagern. Der aufwendigste Schritt im Gesamtablauf, die Belichtung, kann folglich zentralisiert werden, d.h. dem Lagerbetrieb angegliedert werden.

Darüber hinaus wurde überraschend gefunden, daß insbesondere auch solche Früchte, die bei normaler Reifung und Lagerung grün bleiben, z.B. grüne Äpfel, nach einer Behandlung mit UV-B oder einer Mischung aus UV-B und weißem Licht eine kräftige Rotfärbung zeigen.

### Figuren:

Die Figur 1 ist eine schematische Skizze einer Bestrahlungskammer, die sich für das erfindungsgemäße Verfahren eignet.

Die Figur 2 enthält 2 Fotografien von Äpfeln (a: Zitronenapfel, b: Golden Delicious), die nach einer 7-tägigen Bestrahlung mit 4 unterschiedlichen Lichtsorten (UV-A/Weiß, UV-B, Blau/Weiß, UV-B/Weiß) aufgenommen wurden.

Figur 3 ist ein Balkendiagramm, in dem die Absorption bei 527,5 nm von Extrakten aus den Schalen von Pilotäpfeln gegen die Bestrahlungsdauer in Stunden für 2 Versuchsreihen (Anthocyanmessung direkt nach der Bestrahlung; Anthocyanmessung nach Bestrahlung und zusätzlicher 7-tägiger Lagerung) aufgetragen ist.

### Beispiele:

### METHODIK:

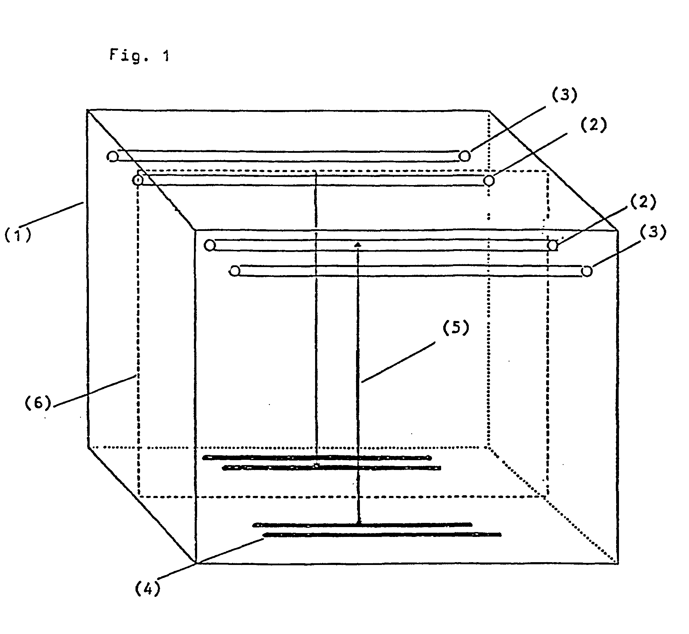

Für das erfindungsgemäße Bestrahlungsverfahren verwendete man eine auf 17°C eingestellte Klimakammer (1), wie sie in Figur 1 dargestellt ist. Beim Arbeiten mit farbigem bzw. UV-Licht wurden je zwei Lampen (2) (Abstand 50 cm, je 25 cm von der Dekkenmitte) in einer Höhe von 80 cm an der Decke einer Klimakammer mit den folgenden Maßen befestigt: 160 cm Höhe x 120 cm Breite x 140 cm Tiefe. Beim Einsatz von Mischungen aus farbigem bzw. UV-Licht mit weißem Licht verwendete man zusätzlich zwei Weißlichtlampen (3), die man rechts und links der farbigen (bzw. UV)-Lampen (2), und zwar jeweils 10 cm weiter von der Deckenmitte entfernt, anbrachte. Für die Bestrahlung mit Weißlicht verwendete man 4 Lampen (2, 3), die die gleiche Anordnung wie bei der Mischlicht-Bestrahlung hatten. Die Position der zu bestrahlenden Früchte (20-50 Früchte pro Versuch) ist durch schwarze Balken (4) dargestellt. Der Abstand zwischen den Früchten und den Lampen ist durch den Doppelpfeil (5) angedeutet. Die Innenwände der gesamten Kammer (1) waren mit einer Metallfolie verspiegelt.

Wo in den Beispielen von einem Versuchsaufbau "halbe Kammer" die Rede ist, wurde die Kammer durch die schwarze, nicht verspiegelte Zwischenwand (6) getrennt. In den Versuchen mit einer "halben Kammer" arbeitete man folglich mit nur 1 bzw. 2 Leuchtstoffröhren und ca. halben Lichtintensitäten.

Die Bestrahlungen wurden mit den folgenden Lichtquellen durchgeführt.
Weißes Licht: Phillips TLD 36W/83 (Länge: 120 cm)
Blaues Licht: Phillips TLD 36W/18 BLUE (Länge: 120 cm)
Rotes Licht: Phillips TLD 36W/15 RED (Länge: 120 cm)
UV-A: Phillips TL 60W/09 N" (Länge: 120 cm)
UV-B: Phillips TL 40W/12 (Länge: 120 cm)

Für die Lampen wurde die in der Tabelle 1 angegebene Strahlungsflußverteilung ermittelt (Abstand ca. 65 cm, jeweils eine Lampe).

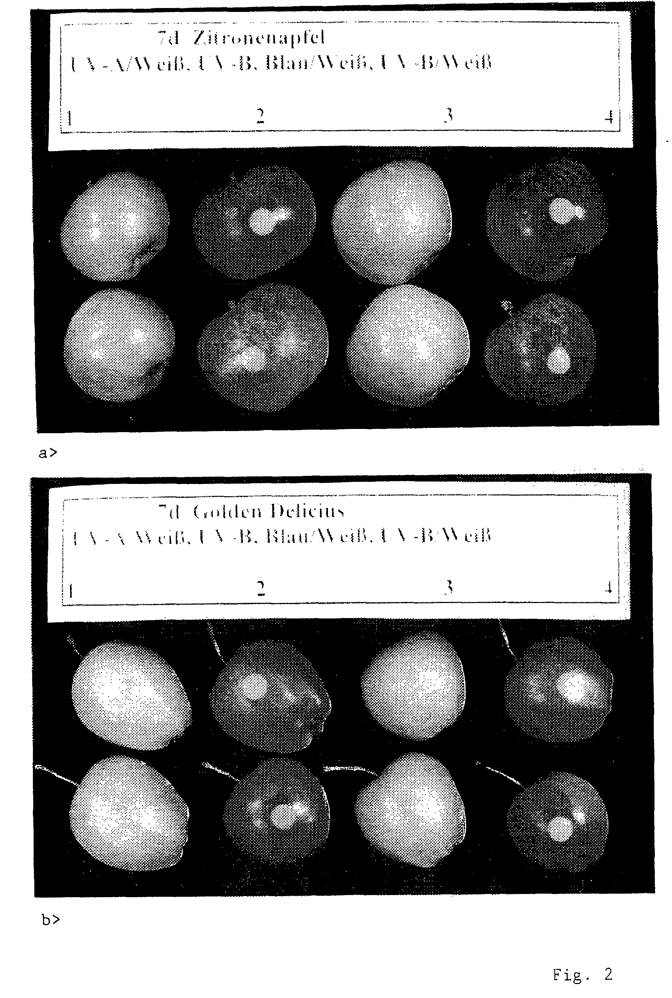

Die Anthocyanbildung wurde über die Zunahme der Rotfärbung der Versuchsfrüchte nach den folgenden drei Verfahren bewertet.
1. Bestimmung der Chromameter-Werte (Y, x, y), wobei Y für Helligkeit, x für Blau-Gelb-Werte und y für Rot-Grün-Werte der Farbe steht. Aus diesen drei Zahlenwerten läßt sich jede Farbe somit wieder "zusammensetzen". Die erhaltenen Werte sind in hohem Maße reproduzierbar. Die Messungen wurden mit einem von der Firma Minolta hergestellten Gerät der Bezeichnung "Chromameter II Reflectance" durchgeführt. Bei der Messung wurde die Versuchsfrucht wie folgt vermessen:
   Der Meßkopf wurde dreimal abgelesen und es wurden Mittelwerte für jeden Meßpunkt angegeben. Die Schwankungen sind ≤ 3% vom Mittel. Die Wertevariation liegt demzufolge in der zwangsläufig nicht vollkommenen Gleichmäßigkeit der Früchte.
2. Photometrische Absorptionsmessungen bei 527,5 nm: nach dem Schälen der Frucht wurden drei kreisförmige Flächen von zusammen 2,4 cm² aus der Fruchtschale mit einem Korkbohrer ausgestanzt, die einmal unter Schütteln mit 1,5 ml einer Mischung aus 10N-HCl und Methanol im Volumen-Verhältnis 1/99 (1% 10N-HCl + 99% MeOH) extrahiert wurden. Dann maß man die Absorption bei 527,5 nm des Extrakts in einer Küvette mit einer Pfadlänge von 1 cm unter Verwendung eines handelsüblichen Photometers, das von der Fa. Perkin-Elmer hergestellt wurde.
3. Optische Bewertung: vor dem Auslegen der Früchte wurden schwarze Klebetikette auf die Schale geklebt, unter denen beim Bestrahlen die ursprüngliche Farbe erhalten blieb. Nach Beendigung der Belichtung wurde auf einem Foto die ursprüngliche mit der direkt daneben entstandenen neuen Farbe verglichen. In den Fig. 2a-2b sind für mehrere der durchgeführten Versuche die entsprechenden Fotografien beigefügt. Bei der optischen Bewertung wurden die Früchte wie folgt klassifiziert:
   "+++": sehr starke Rotfärbung
   "++": starke Rotfärbung
   "+": schwache Rotfärbung
   "-/+": sehr schwache Rotfärbung"
   "-": keine Rotfärbung

Alle drei Methoden liefern völlig übereinstimmende Ergebnisse.

### BEISPIEL 1 (Kinetik)

Frisch geerntete (Ernte Herbst 1997 und 1998) Äpfel der folgenden Sorten wurden vom Versuchsgut der Universität Hannover zur Verfügung gestellt. Die Bestrahlung der folgenden Sorten wurde mit einer "halben Kammer" jeweils 0 bis 5 Tage nach der Ernte durchgeführt (Ausn.: Mutsu wurde in der "ganzen Kammer" untersucht).

Rotwerdende Sorten: *Cox Orange, Elstar, Gloster, Idared, Jonagold, Pilot;*
Gründe Sorten: *Golden Delicious*, *Zitronenapfel*, *Mutsu*

Ferner wurde in Beispiel 1 in einem Versuchsaufbau mit einer "ganzen Kammer" die Rotfärbung von bereits 30-50 Wochen gelagerten *Granny Smith*-Äpfel untersucht, die aus Neuseeland stammten.

Äpfel gleicher Färbung wurden aus einer jeden Sorte ausgewählt. Jeweils ein Apfel wurde dann über einen Zeitraum von 0, 3, 5 bzw. 7 Tagen in der zuvor beschriebenen Klimakammer (17°C) mit Dauerlicht bestrahlt. Als Lichtquellen wurden
- eine Mischung aus blauem Licht und weißem Licht (Erfindung)
- blaues Licht (Vergleich)
- UV-B (Erfindung)
- eine Mischung aus UV-B und weißem Licht (Erfindung)
- UV-A (Vergleich)
- eine Mischung aus UV-A und weißem Licht (Vergleich) eingesetzt. Die Lichtquellen waren wie im Absatz ("Methodik" beschrieben angeordnet.

Unmittelbar nach der Bestrahlung wurde jeder Apfel mit einem Chromameter vermessen. Dann entfernte man mit einem Korkbohrer ein Stück der Apfelschale, die dann nach dem zuvor beschriebenen Verfahren (2) auf ihren relativen Gehalt (bezogen auf die Nullwerte) an Anthocyan untersucht wurde.

Die Werte für "0 Tage" (Nullwert) wurde durch die Untersuchung einer abgeklebten Fläche oder durch Messung zu Versuchsbeginn erhalten.

Die Ergebnisse sind in der Tabelle 2a zusammengestellt. Die Schwankungen der Meßwerte sind durch die niemals perfekte Übereinstimmung zwischen Äpfeln einer Meßreihe bedingt. Ein Teil der Ergebnisse ist in den Fig. 2a-2b dargestellt, die 2 grünen Sorten zeigen (2a: Zitronenapfel, 2b: Golden Delicious), die jeweils 7 Tage mit den folgenden Lichtsorten bestrahlt wurden:
1. UV-A/Weiß, 2: UV-B, 3: Blau/Weiß, 4: UV-B/Weiß

Die Ergebnisse einer weiteren Versuchsreihe unter identischen Bedingungen, in der jedoch ausschließlich eine photometrische Anthocyanmessung nach 0 und 3 Tagen stattfand ist in Tabelle 2b dargestellt.

Die Zunahme roter Anthocyane bei der Bestrahlung mit UV-B oder UV-B/Weiß erkennt man in Fig. 2a und 2b an der Kreisfläche auf den Äpfeln, die dem mit einer lichtundurchlässigen Folie abgedeckten Bereich entspricht, und wo sich somit die Farbe der unbestrahlten Äpfel erhalten hat.

Die Ergebnisse zeigen, daß die Bildung roter Anthocyane durch UV-B-Licht stark gefördert wurde, wobei man die Wirkung durch Beimischung von weißem Licht noch verstärken kann. Gute Ergebnisse erhielt man bei natürlicherweise rot werdenden Sorten auch beim Bestrahlen mit einer Mischung aus weißem und blauem Licht. Die Effekte, die man mit einer Mischung aus weißem und blauem Licht erhält, waren jedoch bis auf wenige Ausnahmen schwächer als beim Bestrahlen mit UV-B-Licht oder beim Bestrahlen mit einer Mischung aus UV-B und weißem Licht, so daß man eine merkliche Rotfärbung erst ab einer Bestrahlungsdauer von mehr als 7 Tagen beobachtete. Blaues Licht allein zeigt eine wesentlich schwächere Wirkung als UV-B oder UV-B/weiß. die Bestrahlung mit rotem Licht und UV-A (mit oder ohne weißes Licht) war nahezu wirkungslos. Da UV-A-und UV-B-Röhren im sichtbaren Bereich ungefähr die gleiche, wenn auch geringere Strahlungsdichte (vgl. Tabelle 1) zeigen, belegt dieses Ergebnis, daß die mit UV-B erzielten Ergebnisse nicht durch "Verunreinigungen" mit weißem oder blauen Licht hervorgerufen werden.

Die Tabellen 2a und 2b (Mutsu, Zitronenapfel und Granny Smith) zeigen auch, daß man bei "grünen" Sorten eine Anthocyanbildung nur mit UV-B-Licht, insbesondere in Mischung mit Weißlicht erzielt.

### BEISPIEL 2 (Bestrahlung und Nachlagerung)

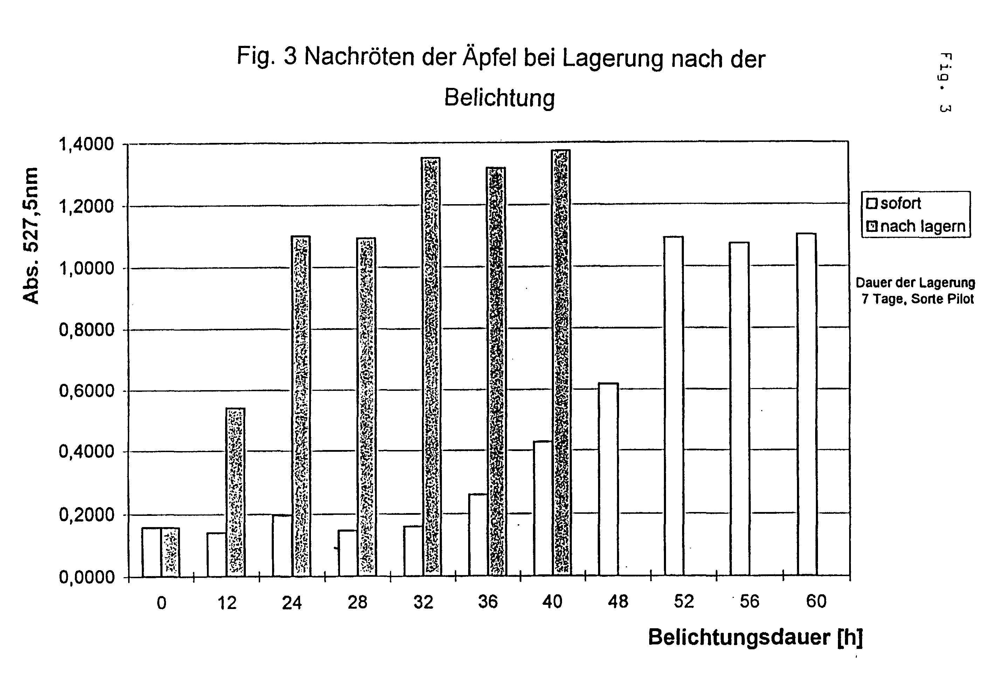

Die Apfelsorten *Cox Orange, Jonagold, Pilot* und *Golden Delicious* wurden unter den in Beispiel 1 angegebenen Bedingungen Oh, 12h, 24h, 32h und 40h mit einer Mischung aus UV-B Licht und weißem Licht in der Klimakammer (17°C) bestrahlt. Die Messung der Anthocanbildung erfolgte in Beispiel 2 nicht nur unmittelbar nach der Bestrahlung, sondern auch nach einer zusätzlichen 7-tägigen Lagerung in einem Kühlschrank (bei 4°C). Die Messung der Anthocyanbildung erfolgte mit den zuvor erläuterten Verfahren (1) und (2). Die Ergebnisse sind in Tabelle 3 zusammengefaßt. Die mit Pilotäpfeln erhaltenen Ergebnisse (und zusätzliche Messungen an Pilotäpfeln nach 28h, 36h, 48h, 52h und 60h) sind in **Fig. 3** graphisch dargestellt. Fig. 3 ist ein Balkendiagramm, in dem die Anthocyanbildung (gemessen durch Absorptionsmessungen bei 527,5 nm) für Pilotäpfel dargestellt sind, bei denen die Messung entweder sofort nach der Bestrahlung oder nach einer zusätzlichen Nachlagerung von einer Woche bei 4°C im Dunkeln vorgenommen wurde. Die beobachteten geringfügigen Schwankungen beruhen darauf, daß die Messungsreihe mit unterschiedlichen Äpfeln vorgenommen werden mußte.

Die Ergebnisse zeigen, daß bei der Bestrahlung mit einer Mischung aus UV-B-Licht und weißem Licht schon 12h genügen, um eine merkliche Anthocyanbildung hervorzurufen.

Der Vergleich mit den entsprechenden Werten des Beispiels 1 zeigt ferner, daß während der Nachlagerung im Dunkeln (4°C) sich weiterhin Anthocyan bildete. Sehr gute Werte erhält man bereits bei einer 24-stündigen Bestrahlung und einer 7-tägigen Nachlagerung.

### BEISPIEL 3 (Lageräpfel):

Frisch geerntete Äpfel der folgenden Sorten wurden bis zu 4 Monaten bei Normalbedingungen oder bis zu 12 Monaten unter ULO-Bedingungen gelagert und dann unter den gleichen Bedingungen wie in Beispiel 1 bestrahlt, abgesehen davon, daß man über eine Dauer von 3 Tagen bzw. 7 Tagen (teilweise 13 Tage) bestrahlte, wobei man je nach Lichtquelle(n) die folgenden Messungen durchführte:
7-Tage-Werte (13 Tage-Werte) für Blau / Blau + Weiß / UV-A, 3-Tage-Werte für UV-B und UV-B + Weiß.
Die Anthocyanbildung wurde nach den Verfahren (1) bis (3) bestimmt.

Rotwerdenden Sorten: *Cox Orange, Elstar, Gloster, Idared, Jonagold, Pilot;*
Grüne Sorten: *Golden Delicious, Granny* Smith und *Mutsu*.

Die (nicht dargestellten) Ergebnisse zeigten, daß auch bei der Bestrahlung von längerer Zeit gelagerten Äpfeln mit einer Mischung aus UV-B und weißem Licht die Rotfärbung bei allen Äpfeln nach drei Tagen voll ausgebildet war.

Die Mischung aus blauem + weißem Licht zeigte eine stärkere Wirkung als bei frisch geernteten Äpfeln. So erhält man im Unterschied zu frisch geernteten Äpfeln (vgl. Tabelle 2) bereits nach 7 Tagen eine merkliche Rotfärbung. Bei einer Bestrahlung mit einer Mischung aus blauem und weißem Licht von gelagerten Idared-Äpfeln erhielt man beispielsweise nach 13 Tagen eine leuchtend rote Färbung, ausgehend von grünen Äpfeln.

Bei schlecht rot werdenden Sorten (z.B. Cox Orange) hat UV-B allein eine stark beschleunigende Wirkung, ebenso wie bei allen anderen Sorten. Bei den ohnehin relativ gut rot werdenden sorten z.B. Pilot und Gloster hat schon blaues Licht (oder eine Mischung aus blauem und weißem) eine beschleunigende Wirkung.

Der Zeitverlauf der Rotfärbung ist bei den gut rot werdenden Sorten nicht wesentlich schneller als bei den grünen Sorten.

Der überraschendste Befund war jedoch, daß alle drei "grünen" Sorten mit UV-B allein, oder noch stärker, mit UV-B + weiß, ebenfalls vollständig rot werden.

### BEISPIEL 4 (andere Früchte):

Gelagerte, noch gründe Birnen der Sorte Abate (Herkunftsland: Italien) wurden auf die gleiche Weise wie in Beispiel 1 bestrahlt, jedoch nur mit UV-B-Licht, UV-B- und Weißlicht, Blaulicht und Blau- und Weißlicht. Nur UV-B- bzw. UV-B und Weißlicht führten zu einer Rötung der Früchte nach 3 bis 7 Tagen.

Dieses Ergebnis zeigt, daß das erfindungsgemäße Verfahren auch auf andere Früchte als Äpfel anzuwenden ist.

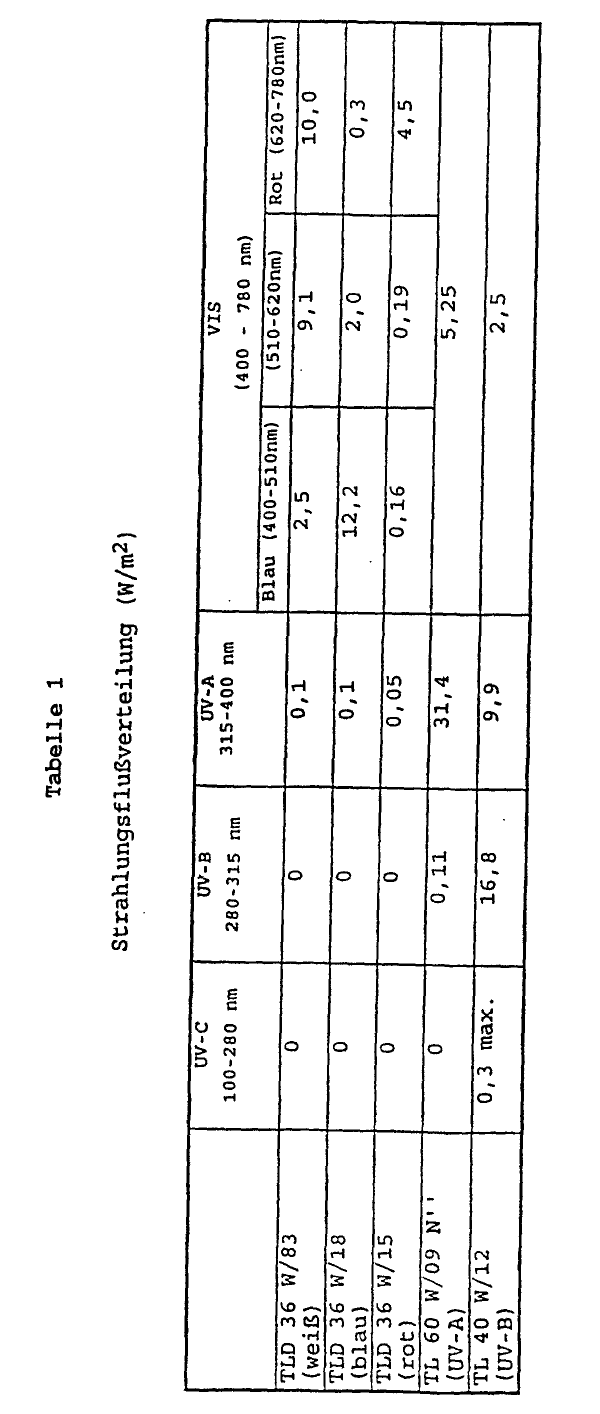

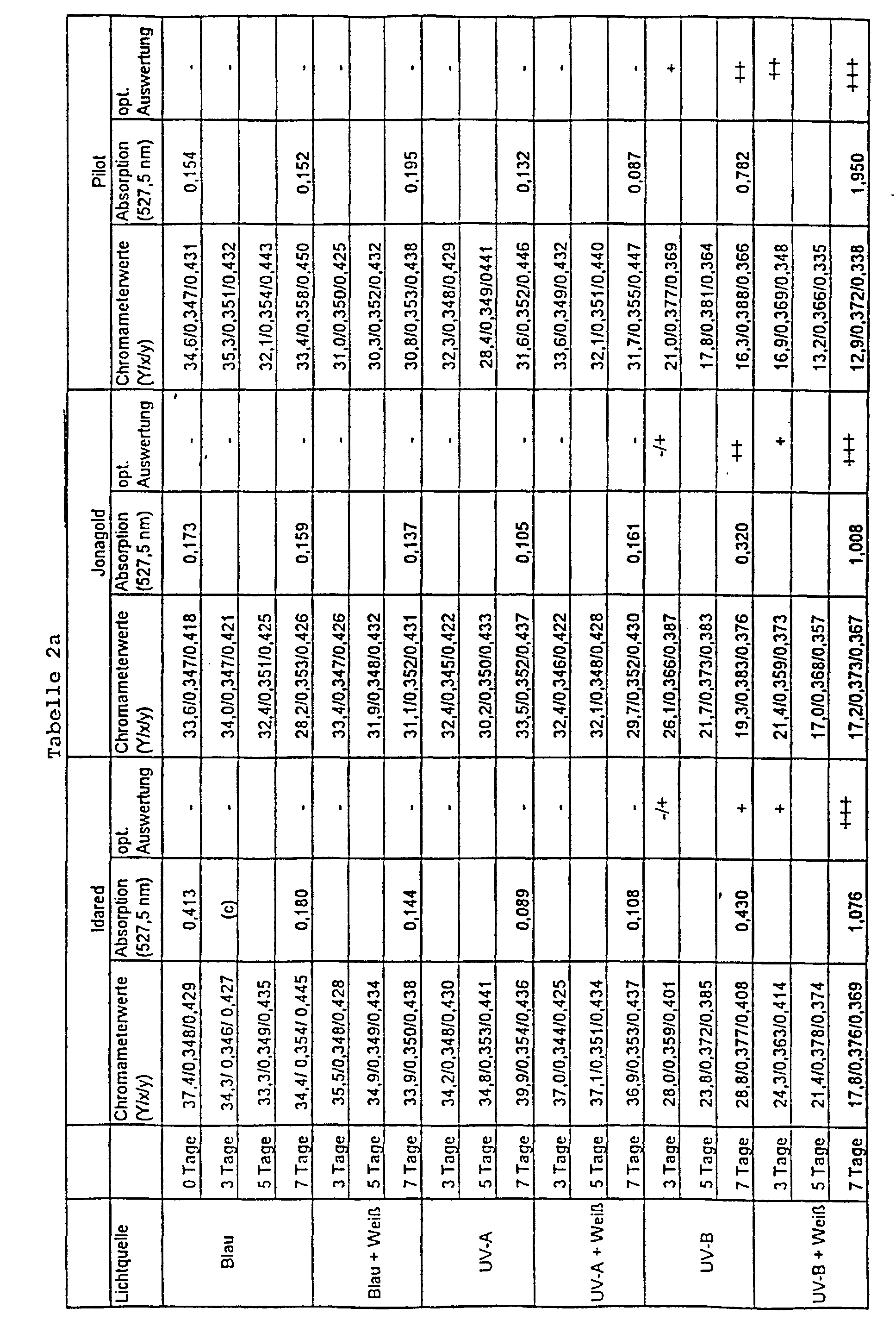

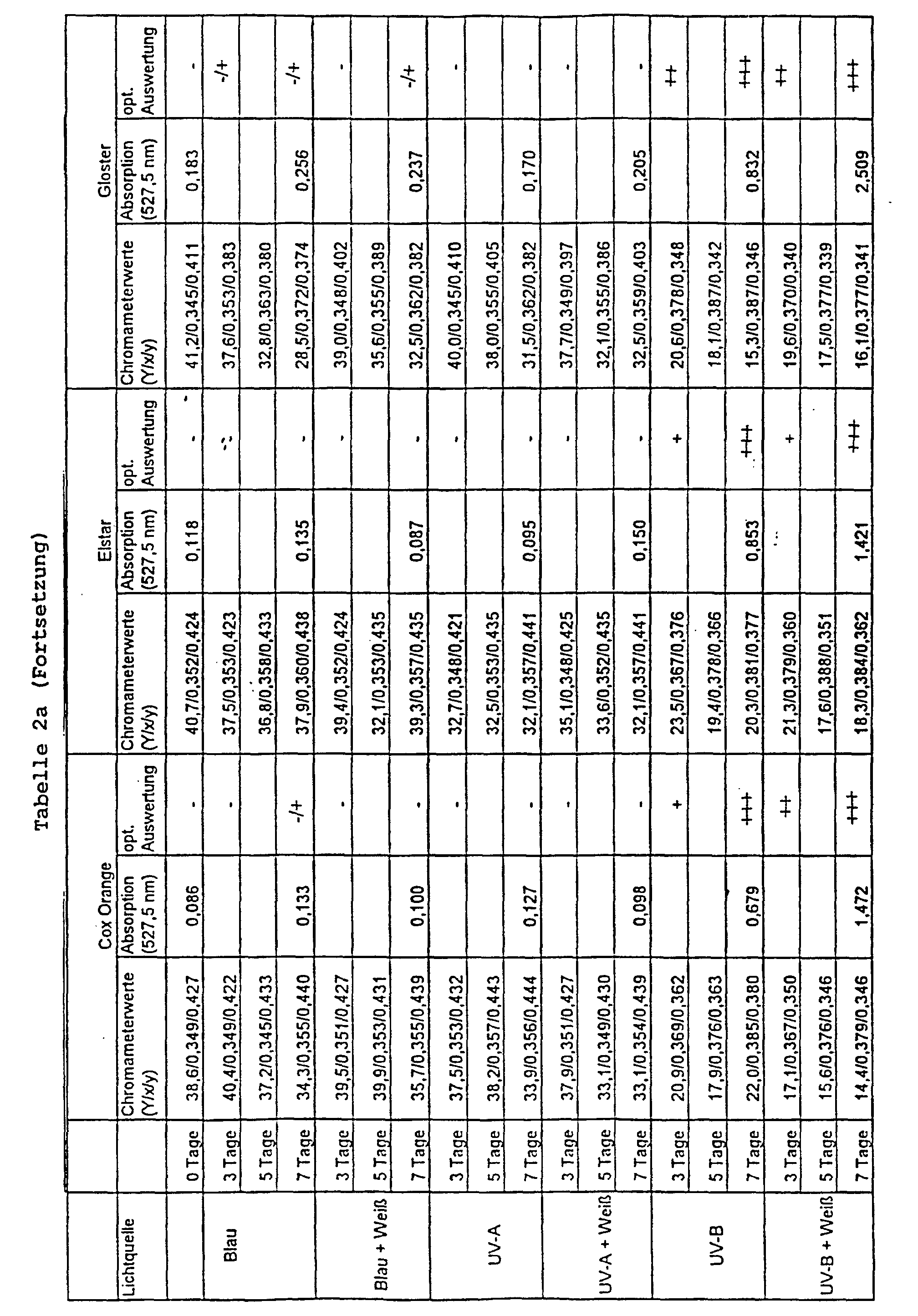

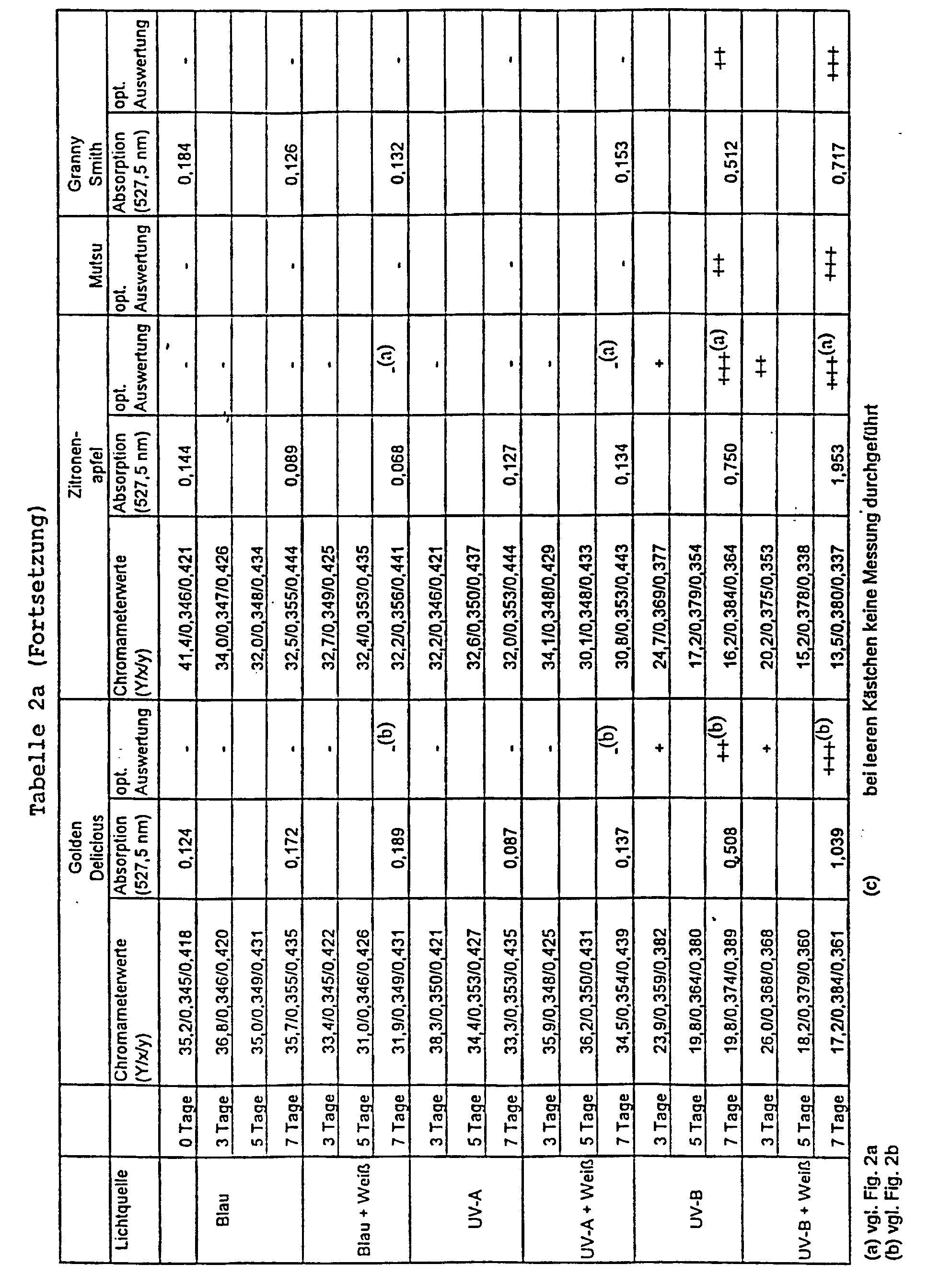

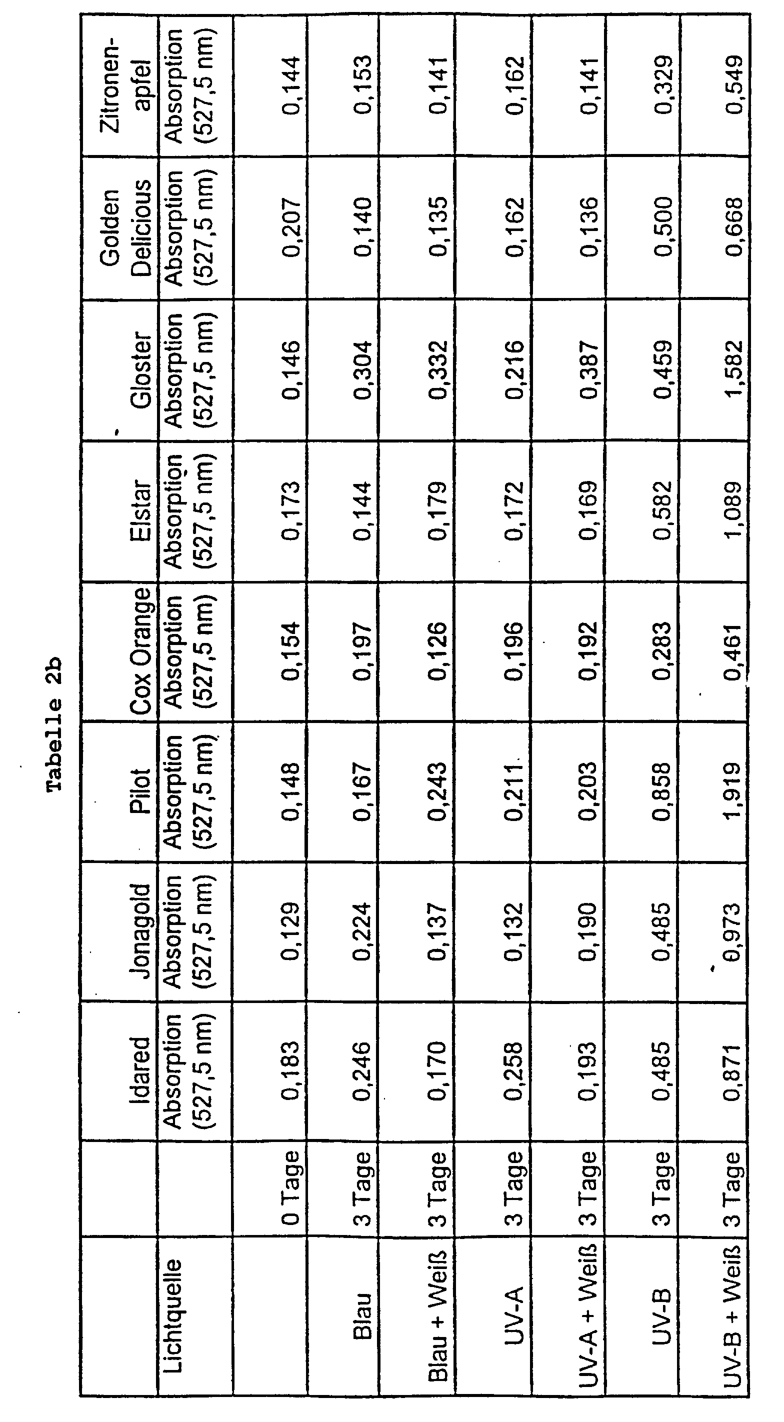

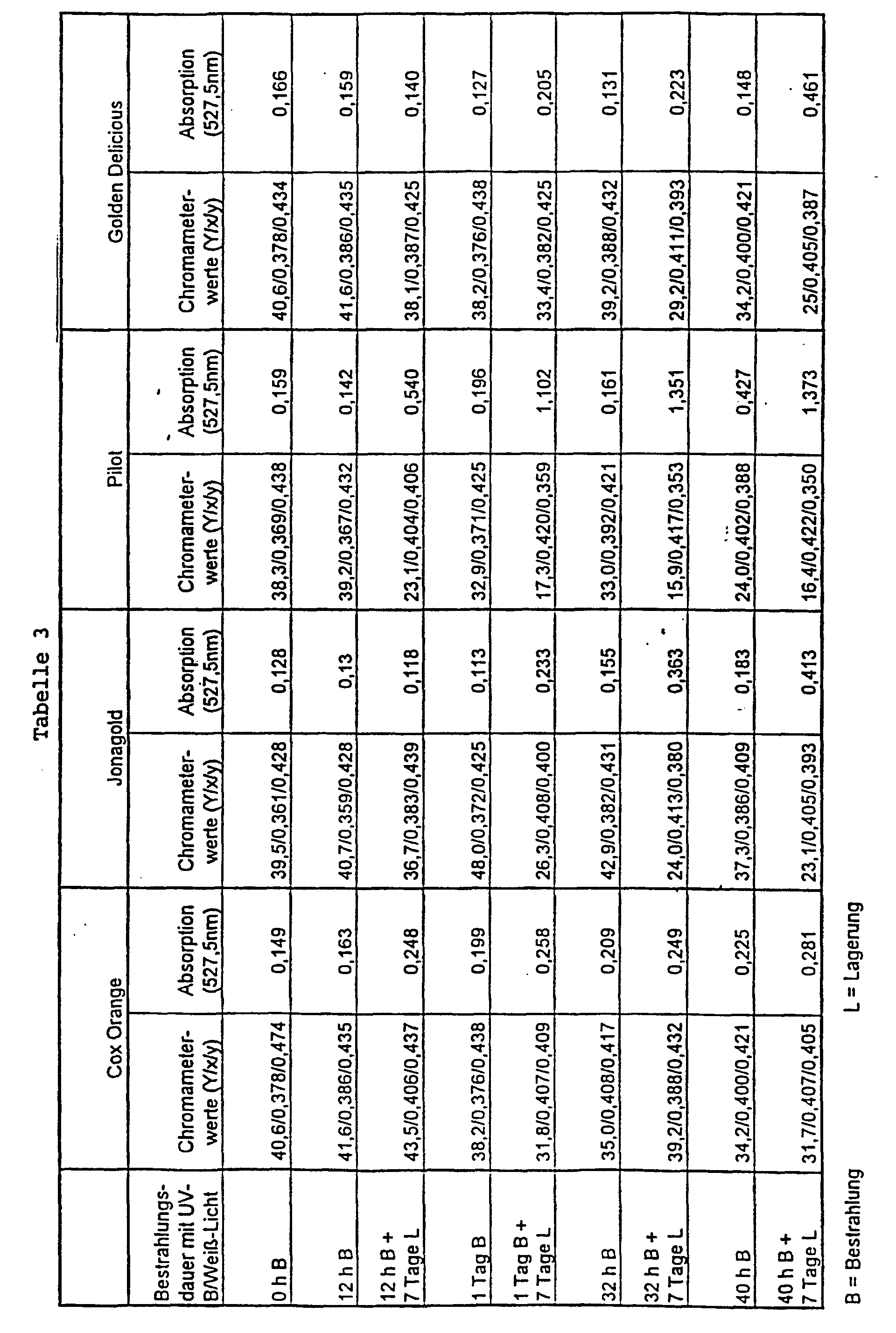

## Patentansprüche

1. Verfahren zum Hervorrufen einer Rotfärbung durch Anthocyan an grundsätzlich Anthocyan-bildenden Pflanzen und/oder Früchten, die natürlicherweise beim Reifen nicht rot werden, durch Bestrahlung der Pflanzen und/oder Früchte mit UV-B-Licht oder einer Mischung aus UV-B und weißem Licht.

2. Verfahren nach Anspruch 1, **dadurch gekennzeichnet, daß** die Bestrahlung bezogen auf die Wattleistung wenigstens 10% Licht im Wellenlängenbereich zwischen 280 und 315 nm UV-B-Licht enthält, vorzugsweise wenigstens 20%.

3. Verfahren nach Anspruch 1 oder 2, **dadurch gekennzeichnet, daß** die Früchte aus Äpfeln und Birnen ausgewählt werden.

4. Verfahren nach Anspruch 3, **dadurch gekennzeichnet, daß** die Äpfel vorzugsweise ausgewählt sind aus den Sorten *Golden Delicious, Zitronenapfel, Granny Smith* und *Mutsu.*

5. Verfahren nach einem der Ansprüche 1 bis 4, **dadurch gekennzeichnet, daß** man die Pflanzen und/oder Früchte über eine Dauer zwischen 6 Stunden und mehreren Tagen, vorzugsweise zwischen 12 h und 72 h bestrahlt.

6. Verfahren nach einem der Ansprüche 1 bis 5, **dadurch gekennzeichnet, daß** man die Bestrahlung bei einer Temperatur von 0 bis 30°C, vorzugsweise 5 bis 25°C durchführt.

7. Verfahren nach einem der Ansprüche 1 bis 6, **dadurch gekennzeichnet, daß** der Abstand der zu bestrahlenden Pflanzen und/oder Früchte zu der oder den Lichtquellen bis zu 3 m, vorzugsweise 25 bis 100 cm beträgt.

8. Verfahren nach einem der Ansprüche 1 bis 7, **dadurch gekennzeichnet, daß** man die Früchte nach der Bestrahlung im Dunkeln lagert.

9. Verfahren nach Anspruch 8, **dadurch gekennzeichnet, daß** man über eine Dauer von 12 bis 72 h bestrahlt und anschließend bei 0-10°C für mindestens 2 Tage im Dunkeln lagert.

10. Verfahren nach Anspruch 8 oder 9, **dadurch gekennzeichnet, daß** die Früchte nach der Bestrahlung in einem ULO-Lager oder CA-Lager gelagert werden.

11. Verfahren nach einem der Ansprüche 1 bis 10, **dadurch gekennzeichnet, daß** man zur Ausbildung einer Aussparung der Anthocyanfärbung mit einer beliebig gewählten Form vor der Bestrahlung eine lichtundurchlässige Abdeckung mit dieser Form auf die nicht oder wenig gefärbten Pflanzen und/oder Früchte aufbringt und die Abdeckung nach der Bestrahlung wieder entfernt.

12. Pflanze und/oder Frucht einer natürlicherweise nicht rotwerdenden Sorte, die eine Anthocyanrotfärbung aufweist, erhältlich nach einem Verfahren gemäß einem der Ansprüche 1 bis 11.

## Claims

1. Method for provoking a red colouration by anthocyanin in plants and/or fruits which form anthocyanin in principle but do not naturally turn red upon maturation, by irradiating the plants and/or fruits with UV-B light or a mixture of UV-B and white light.

2. Method according to Claim 1, **characterized in that** the irradiation comprises at least 10% light in the wavelength range between 280 and 315 nm UV-B light, preferably at least 20%, based on the active output.

3. Method according to Claim 1 or 2, **characterized in that** the fruits are selected from amongst apples and pears.

4. Method according to Claim 3, **characterized in that** the apples are preferably selected from amongst the cultivars *Golden Delicious, Zitronenapfel, Granny Smith* and *Mutsu.*

5. Method according to one of Claims 1 to 4, **characterized in that** the plants and/or fruits are irradiated over a period of between 6 hours and several days, preferably between 12 hours and 72 hours.

6. Method according to one of Claims 1 to 5, **characterized in that** the irradiation is carried out at a temperature of from 0 to 30°C, preferably 5 to 25°C.

7. Method according to one of Claims 1 to 6, **characterized in that** the distance between the plants and/or fruits to be irradiated and the light source(s) amounts to up to 3 m, preferably 25 to 100 cm.

8. Method according to one of Claims 1 to 7, **characterized in that** the fruits are stored in the dark post-irradiation.

9. Method according to Claim 8, **characterized in that** the irradiation is carried out over a period of from 12 to 72 hours, followed by storage in the dark at 0-10°C for at least 2 days.

10. Method according to Claim 8 or 9, **characterized in that** the fruits are stored in a ULO store or CA store post-irradiation.

11. Method according to one of Claims 1 to 10, **characterized in that**, to develop an area of any desired shape which lacks anthocyanin colouration, a mask in this shape which is impervious to light is placed on the uncoloured or sparingly coloured plants and/or fruits and the mask is removed post-irradiation.

12. Plant and/or fruit of a variety which does not naturally turn red but with red colouration due to anthocyanin, obtainable by a method according to one of Claims 1 to 11.

## Revendications

1. Procédé pour induire une coloration rouge par l'anthocyane dans des plantes et/ou des fruits qui engendrent par principe de l'anthocyane et qui, lors de leur mûrissement, naturellement, ne deviennent pas rouges, en soumettant les plantes et/ou les fruits à un rayonnement de lumière UV-B ou un mélange de lumière UV-B et de lumière blanche.

2. Procédé selon la revendication 1, **caractérisé en ce que** le rayonnement comprend, par rapport à sa puissance en Watts au moins 10% de lumière UV-B dans l'intervalle de longueur d'onde compris entre 280 et 315 nm, de préférence au moins 20%.

3. Procédé selon la revendication 1 ou 2, **caractérisé en ce que** les fruits sont choisis parmi les pommes et les poires.

4. Procédé selon la revendication 3, **caractérisé en ce que** les pommes sont choisies de préférence parmi les espèces Golden Delicious, les pommes citron, les Granny Smith et Mutsu.

5. Procédé selon l'une des revendications 1 à 4, **caractérisé en ce qu'**on soumet les plantes et/ou les fruits à un rayonnement pendant une durée comprise entre 6 h et plusieurs jours, de préférence pendant 12 h à 72 h.

6. Procédé selon l'une des revendications 1 à 5, **caractérisé en ce que** le rayonnement est effectué à une température de 0 à 30° C, de préférence de 5 à 25° C.

7. Procédé selon l'une des revendications 1 à 6, **caractérisé en ce que** la distance entre les plantes et/ou les fruits à soumettre au rayonnement et la ou les sources de lumière atteint jusqu'à 3 m et est comprise de préférence entre 25 et 100 cm.

8. Procédé selon l'une des revendications 1 à 7, **caractérisé en ce que** les fruits sont, après leur exposition au rayonnement, stockés dans un endroit sombre.

9. Procédé selon la revendication 8, **caractérisé en ce qu'**on soumet au rayonnement pendant une durée de 12 à 72 h et ensuite on stocke dans un endroit sombre à 0-10° C pendant au moins deux jours.

10. Procédé selon la revendication 8 ou 9, **caractérisé en ce que** les fruits, après exposition au rayonnement, sont stockés dans un magasin ULO ou CA.

11. Procédé selon l'une des revendications 1 à 10, **caractérisé en ce que** pour éviter la coloration de l'anthocyane suivant une forme choisie prédéterminée, on recouvre avant l'exposition au rayonnement par un revêtement ayant cette forme, imperméable à la lumière, les plantes et/ou fruits non ou peu colorés et on retire le revêtement après exposition à la lumière.

12. Plantes et/ou fruits d'une espèce qui ne devient pas naturellement rouge et présente une coloration de l'anthocyane, pouvant être obtenus par un procédé selon l'une des revendications 1 à 11.
